# EUROPEAN PATENT APPLICATION

(11) **EP 3 796 236 A1**
(43) Date of publication of application: **24.03.2021**
(21) Application number: 19306121.5
(22) Date of filing: 18.09.2019
(51) Int. Cl.: G06N 5/02, G06F 16/36, G16H 10/60

(54) **MODELING AND ANALYSIS OF COMPLEX SYSTEMS**

(71) Applicant: Vieviewer Entreprise, 92400 Malakoff (FR)
(72) Inventor: Fouladi, Karan, 92400 Malakoff (FR); Djavahery, Djavad, 92400 Malakoff (FR)
(74) Representative: Grünecker Patent- und Rechtsanwälte PartG mbB

(57) **Abstract**

The invention relates to a computer-implemented method for modeling a complex system, CS, the method comprising receiving heterogeneous raw data representing the CS, applying rules and constraints from one or more domain ontologies to the heterogeneous raw data to obtain a corpus of categorized and terminologically indexed data, applying a first set of algorithms to the corpus to obtain a plurality of conceptual ontological graphs, COGs, wherein each of the plurality of COGs is categorized as either a state or an event, applying a second set of algorithms to the COGs to identify a chronological dependence of the classified states and events, and to identify a context for each of the classified states and events, applying a third set of algorithms to the COGs to consolidate the classified states and events such that the CS is characterized by one or more periods, wherein one period is characterized by a consolidated state and one or more events associated with the consolidated state, in order to generate a knowledge base of the CS, applying a fourth set of algorithms to the knowledge base to obtain at least one hypothesis of how at least one period of the CS relates to at least one event, generating, using the knowledge base, the at least one period, and the at least one hypothesis, a chronological graphical representation of the complex system, presenting the chronological graphical representation of the complex system and the at least one hypothesis to a user, receiving interaction with and feedback on at least the at least one period and at least one hypothesis from the user, and applying a fifth set of algorithms to the feedback received from the user and the chronological graphical representation of the complex system to generate an updated knowledge base, an updated chronological graphical representation, and an updated hypothesis of the complex system.

## Description

### Field of the invention

The invention relates to a computer-implemented method and a system for the modeling, analysis and management of complex systems.

### Background

Complex systems are systems composed of individual components, which interact with each other. Non-exhaustive examples of complex systems are the Earth's global climate, living cells, the human body, ecosystems, financial markets, and social and economic organizations (like cities). The behavior of complex systems is intrinsically difficult to model due to the dependencies, competitions, relationships, or other types of interactions between their parts or between a given system and its environment. In particular, complex systems have distinct properties that arise from these relationships, such as nonlinearity, emergence, spontaneous order, and adaptation, feedback loops in which small changes can have striking effects, and their evolution in time among one another.

A particularly illustrative and important example of a complex system is a human patient. A patient's medical history and, ultimately, their current state of health can be considered to be the result of the interaction of many individually interacting parts. Starting with the patient's birth (or even with the pre-natal conditions in their mother's womb), continuing through childhood, adolescence and adulthood, every illness, accident or medical treatment that the patient has experienced may contribute to their current state of health. In addition, their living circumstances, such as nutrition, environmental exposure, and so on, may all contribute to their current state of health.

As such, all these factors should usually be considered together with a patient's symptoms and signs when performing a diagnosis of a patient. However, such a task can be difficult in view of the sheer number of parameters and interacting parts that form the complex system that is the patient. In particular in cases where the patient presents with symptoms that can be caused by a number of different conditions, i.e., where it is important to perform a differential diagnosis based on signs, all parts of the complex system that is the patient have to be considered. While this is generally well-known, it is still a difficult task, usually reserved for specialists, which is time-consuming and expensive.

The same applies to other types of complex systems as mentioned above - their analysis is difficult and time-consuming, even for experts in their respective fields. Conclusions as to the state of the complex system are sometimes based more on the experience or "gut feeling" of the expert instead of a detailed analysis and synthesis of the system, simply because such a process would be too costly in terms of time and resources.

So-called "knowledge systems" have been used in the past, first as expert systems, where an inference component of the system applies a set of logic rules to a knowledge base in order to assert new knowledge into the knowledge base and obtain a result from it. These inference engines evolved and include explanatory capabilities, i.e., the capability to explain to a user the chain of reasoning and to take into account the evolution and emergence of complex systems.

However, in particular the task of obtaining the data and forming the knowledge base has been a difficult task in legacy systems.

For these reasons, an object of the invention is to provide a method and systems that makes the modeling and analysis of complex systems more manageable and effective in order to manage them.

### Summary of the invention

This object is achieved with a computer-implemented method according to claim 1. Preferred embodiments are specified in the dependent claims.

In a first step, a computing system executing this method receives heterogeneous raw data representing the complex system. Here, the term "heterogeneous raw data" describes data that comes from various and different data sources. The heterogeneous raw data has no overarching data structure that is common to all elements of the data. In the heterogeneous raw data, the nomenclature of the data may also vary between data elements. Examples of heterogeneous raw data are patient medical data from different medical practitioners, test results, previous prescriptions, et cetera. In particular, the heterogeneous raw data may be considered to represent an unsorted and redundancy collection of available information about the complex system. The heterogeneous raw data may be received in a variety of ways. Parts of it may be retrieved electronically from a network storage. Parts of it may be entered manually into the computing system. Parts of it may be scanned or otherwise imported into the computing system. All available heterogeneous raw data may be received together, or it may be received in parts.

In a next step, the system applies rules and constraints from one or more domain ontologies to the heterogeneous raw data to obtain a corpus of categorized and terminologically indexed data. The use of domain ontologies is a powerful tool in particular for recognizing semantic correspondence of similar elements in heterogeneous raw data in order to consolidate them. This way, parts of the heterogeneous raw data that are similar or related can be recognized and indexed as such. In the example of the medical data indicated above, this corresponds to introducing a consistent terminology and relational structure throughout the medical data based on the domain ontologies. Further, it includes assigning data to different categories, for example, "prescribed medication", "accidents", "illnesses", et cetera. Finally, this step involves creating an index of the data that has been categorized and provided with a homogeneous terminology.

In the following, reference will be made to several different sets of algorithms that are used by the system. Some or all of these sets may at least partly be based on Peirce's Abductive reasoning logic. Here, "Peirce's Abductive reasoning logic" refers to the logical concept developed by Charles Sanders Peirce in the late 19^{th} and early 20^{th} century. It is noted that this is often referred to as "retroductive" reasoning, as both terms were used by Peirce synonymously [Charles Sanders Peirce, Le Raisonnement et la logique des choses, Paris, Cerf, Hilary Putnam des conferences de Harvard en 1896, 1994, https://www.editionsducerf.fr/librairie/livre/7788/raisonnement-et-la-logique-des-choses-le].

Subsequently to creating the index of the data, the system applies a first set of algorithms in order to obtain a plurality of conceptual ontological graphs, COGs, wherein each of the plurality of COGs is categorized as either a state or an event.

For example, to achieve this, a first-order logic algorithm may first be applied to the corpus of categorized and terminologically indexed data in order to obtain "snippets" of knowledge in the form of conceptual graphs. These conceptual graphs may then be compared with each other in order to be consolidated and to build conceptual ontological graphs. Advantageously, a weighted bipartite matching algorithm may be used for the comparison of the conceptual graphs. Such an algorithm may implemented in a computer program in a known manner.

In particular, the first set of algorithms may be based on Sowa's distinction of processes [Sowa: Sowa, John F. (2000) Knowledge Representation: Logical, Philosophical, and Computational Foundations, Brooks Cole Publishing Co., Pacific Grove, CA., http://www.jfsowa.com/krbook/index.htm and http://www.jfsowa.com/ontology/process.htm]. In particular, the system may apply the first set of algorithms to construct two types of COGs according to the "Discrete" branch as defined by Sowa, i.e., "Event" and "State". It is noted that system may further apply the first set of algorithms to infer, using the "Continuous" branch as defined by Sowa, temporal concepts and add a respective temporal concept to each COG Event or State. These COGs and their respective additional concepts (i.e., temporal concepts and contextual concepts) may be used to specify the evolution and context of meaning of the complex systems in following steps.

COGs of the Event or State type are well-suited to represent individual elements of the corpus of categorized and terminologically indexed data. In particular, it is an inherent property of graphs that they can represent not only singular elements, but that they can also be connected. In other words, they are useful to represent elements that can interact with each other. Classifying each COG as either an event or a state serves to further differentiate the COGs as the building blocks of the complex system.

A second set of algorithms is applied to the COGs to identify a chronological dependence of the classified states and events. In other words, the COGs are assigned on a "timeline" of the complex system that builds on the chronological concepts of the COGs. The COGs are further assigned to an identified context. Such a context could, for example, be "medical treatment".

In particular, applying the second set of algorithms to the COGs allows extraction of contextual elements to construct a concept dedicated to the context to add to the events and states as well as chronological concepts. This is important, since the meaning of each event and state is based on their own respective context.

The second algorithm may further be applied to acts and interactions of a user of the system that have been tracked with regard to the complex system. Here, the term act specifically refers to actions that the user has taken with respect to the complex system. Such acts could, for example, be the generation of reports, diagnostics and/or conclusions regarding the complex system. Such acts may also be referred to as "descriptions". Acts may also be decisions, orders, recommendations or the like concerning the complex system. Such acts may also be referred to as "prescriptions". Acts may be tracked by the system as they are entered directly into the system or obtained from outside data sources.

On the other hand, the term interactions specifically refers to interactions of the user with a graphical representation of the complex system as displayed by the system. Interactions may, for example, writing observations, accessing and viewing documents, arranging and filtering documents, searching and analyzing the content of descriptions, or the like. The interactions may in particular be logged in a log file.

The tracked user acts, interactions and the context identified may further be used to generate contextual concepts and a pattern of the user's acts and interactions with the complex system. The pattern may be used in the generation of a user model.

In particular, the second set of algorithms may be based on Allen's interval algebra [Allen, J.F.: Maintaining knowledge about temporal intervals. Communication of ACM pp. 832-843 (1983), http://ceur-ws.org/Vol-1656/paper6.pdf] and Peirce's Abductive reasoning logic, in particular abductive behavior model extraction [Yihui Ren et al. Generative Modeling of Human Behavior and Social Interactions Using Abductive Analysis, 2018, https://doi.org/10.1109/ASONAM.2018.8508282].

After this, a third set of algorithms is applied to the COGs to consolidate the classified states and events such that the complex system is characterized by one or more periods. Each of the one or more periods is characterized by a consolidated state and one or more events associated with the consolidated state. In particular, the start and/or end points of one or more of the periods may be associated with one or more events. It is possible that the third set of algorithm identifies the start point of a period to be chronologically earlier than the event associated with the start point of the period. In particular, a change of state of the system implies the beginning of a new period and the end of the previous one. When a period is constructed, its similar states will be consolidated into a state characterizing the period. The third set of algorithms may in particular further be based on Bayesian, fuzzy, and Allen's logic.

For example, in the case of a patient, a period of the complex system could be in "childhood" or "adolescence", and the associated events might be illnesses, accidents, treatments, observed symptoms, etc., which the patient was subject to in that period. In that case, the start point of the period "adulthood" would not exactly be determined by the age of a patient but associated with an event which occurred during this period. While the separation between the periods "childhood", "adolescent" and "adult" may not coincide exactly with these periods when generally defined via age, it may reflect them closely if changes in the patient's state of health allow.

An illness could be associated with the start point of another period. In that case, the events indicate the start point of the period and the diagnosis of the illness. However, it is possible that by applying the third set of algorithms, it is determined that the patient has been ill some time previous to the diagnosis. In this case, the start point of the period will be the time when, according to the third set of algorithms, the patient actually contracted the illness (i.e., the change of state).

With this consolidation, a knowledge base, in particular in chronological form, of the complex system is generated. This knowledge base may be the equivalent of a knowledge base in the prior art systems. The knowledge base may be an ontological knowledge base, in particular an evolutionary ontological knowledge base. The knowledge base may be adapted to auto-update in real time.

A knowledge base build by COGs can advantageously infer new knowledge and anticipate the state of being in the evolution of complex systems, thanks to the formal organization of the constituent blocks of COGs.

Then, a fourth set of algorithms is applied to the knowledge base to obtain at least one hypothesis, in particular a hypothetical state, of how at least one period of the complex system relates to at least to this state, in particular describing the evolutionary development of the complex system. In other words, the system analyzes the COGs associated with the period (events & states) in order to formulate a hypothesis of how at least one consolidated state COG is related to the period. In an example, the system may analyze the COGs of a patient's complex system and derive how a particular event, like an illness, contributed to the state that represents the period, and if a state COG reveals it in particular.

The fourth set of algorithms may, in particular, be based on Peirce's Abductive reasoning logic, and on Bayesian-fuzzy logic and abductive conceptual graphic projection and abductive behavior model extraction [David Poole, Representing diagnostic knowledge for probabilistic Horn abduction, 1991, https://www.researchgate.net/publication/220814605_Representing_Diagnostic_Knowledge_for _Probabilistic_Horn_Abduction]

Using the knowledge base, the at least one period, and the at least one hypothesis, a chronological graphical representation of the complex system is then generated. This may be, for example, in the form of a "timeline" of the complex system, with the identified periods and the events indicated in the form of markers. Interdependencies between events may be shown as well. The chronological graphical representation of the complex system and the at least one hypothesis is then presented to a user.

After this, interaction with and feedback on the at least one period and at least one hypothesis is received from the user, and a fifth set of algorithms is applied to the feedback received from the user and the chronological graphical representation of the complex system to generate an updated knowledge base, an updated chronological graphical representation and an updated hypothesis of the complex system. The feedback may, for example, confirm the at least hypothesis or reject it. It may also refine the hypothesis via abductive reasoning.

The fifth set of algorithms may, in particular, further be based on abductive reinforcement learning concepts [Alexey Ignatiev et al, Abduction-Based Explanations for Machine Learning Models, 2018, https://arxiv.org/abs/1811.10656]

The feedback may be received by interacting with the chronological graphical representation in a graphical user interface. The feedback may be provided through selection of user interface elements, entering of text via a keyboard, and/or through voice commands, accessing and viewing documents, arranging and filtering documents, and/or performing searches. The feedback may also comprise also acts as described above, for example, by analyzing the content of descriptions issuing of prescriptions. The feedback may also comprise new data describing the complex system. In particular, the data may comprise new heterogeneous raw data. The new data may be then used in the generation of the updated hypothesis.

The feedback may further be used in the generation of the above-mentioned user model. The feedback may further be used to update an existing user model. In particular, this may be accomplished by the application of the fifth set of algorithms as specified above.

The system may use the feedback, via machine learning methods, in order to infer and update the user model. In particular, the user model may be directly involved in the update of the knowledge base and hypothetical states, and how they are presented on the graphical user interface. For example, the system may learn from the data representing one patient and apply this to the data representing another patient.

The process of gathering and processing the user's interactions and actions occurs automatically. In particular, the user will not be aware which actions and interactions are gathered and processed. In other words, the user does not know which actions and interactions influence the construction or update of the user model. This way, the user model will more accurately reflect the user, since the user will not be able to bias the model through conscious decisions. The processing of the acts and interactions of the user by the system may be implemented similar to how it is described in [Lim, J., Kim, M., Lee, B. et al. "A target advertisement system based on TV viewer's profile reasoning, 2008" Multimed. Tools Appl. (2008) 36: 11, https://doi.org/10.1007/s11042-006-0079-2].As mentioned above, in order to infer and update the user model, two types of user feedback may be used: acts as descriptions and prescriptions and user interactions with the interface. Here, in particular, the interactions may be considered as the premises and the acts may be considered as the conclusions in an abductive reasoning process to infer a hypothetical user model or update it.

In particular, the main inference process used to abductive reasoning may be Reinforcement Concept Learning as described in [Sutton, R.S. Barto, A.G. Reinforcement Learning-An Introduction, Second Edition, 2017, http://incompleteideas.net/book/bookdraft2017nov5.pdf] and [Karan Fouladi, Recommandation multidimensionnelle d'emissions télévisées par apprentissage, Une interface de visualisation intelligente pour la television numerique, These de doctorat, 2013, http://www.theses.fr/2013PA066040].

By homogenizing the available data about the complex system, putting it into a well-structured form by way of COGs, and then analyzing the COGs as described above, interactions in the complex system can be modeled and analyzed quickly and efficiently, and a hypothesis about the state of the complex system can be generated. Receiving and taking into account feedback on the hypothesis results in a refinement that ultimately leads to an accurate model of the complex system. In the example of a patient, this means that a diagnosis, and therefore a possible choice of treatment, can be found in an efficient manner [Jean Charlet, Gunnar Declerck, Ferdinand Dhombres, Pierre Gayet, Patrick Miroux, et al.. Construire une ontologie médicale pour la recherche d'information : problematiques terminologiques et de modelisation. 23es journées francophones d'Ingénierie des connaissances, Jun 2012, https://hal.archives-ouvertes.fr/hal-00717807/document, Paris, France.pp.33-48. hal-00717807].

The method may further comprise generating a user behavioral model, UBM, based on the interactions of the user with the complex system similar as described in [Jose Hernandez-Orallo, Constructive Reinforcement Learning, 2000, https://doi.org/10.1002/(SICI)1098-111X(200003)15:3%3c241::AID-INT6%3e3.0.CO;2-Z].

A first part of the user actions with respect to the complex system, also referred to as "User Acts Elements", may be extracted from the COGs of the complex system. For example, actions performed by a medical practitioner in relation with a patient may comprise User Acts Elements in the categories "Analysis" (e.g., questions, requests, reflections), "Descriptions" (e.g., reports, diagnostics, conclusions), "Prescriptions" (e.g., decisions, orders, recommendations) which will generally be found in the raw heterogeneous data and as such be reflected in the COGs. These User Acts Elements are then extracted from the COGs. This step may also comprise generating a profile for the user.

A second part of the user actions with respect to the complex system, also referred to as "User Interaction Elements", may be obtained by the system analyzing the interactions of the user with the chronological graphical representation of the complex system and apply one or more of the above-identified sets of algorithms to the interactions of the user. This may comprise updating the knowledge base of the complex system. It may also comprise generating a model for the user. The User Interaction Elements may, e.g., be extracted from log files. The User Interaction Elements may comprise actions in a User Interface, such as writing observations, opening/closing (e.g., records, database, e-mails), consulting sources (e.g., intranet, ontologies, domain information), reading/sending (e.g., reports, documents, acts, e-mails), tuning of the interface (e.g., icon positions, add/filter elements). The User Interaction Elements may be extracted by a specific module from the interactions of the user with the chronological graphical representation of the complex system.

The method may further comprise adapting the chronological graphical representation of the complex system based on the generated or updated UBM. For example, the graphical representation may be updated by highlighting certain periods and/or events of the complex system based on the generated or updated UBM.

The method may further comprise generating at least one recommendation for interaction of the user with a current state of the complex system, based on the updated hypothesis and the generated or updated UBM. In particular, the recommendation may suggest further analysis and/or direct interaction with the complex system. For example, a suggested further analysis may be a recommendation of a further diagnostic test of a patient. A direct act may be a suggestion for the treatment of a patient.

The method may further comprise generating at least one decision making help concerning at least one decision concerning at least one hypothesis, and updating the knowledge base of the complex system.

The complex system may be a patient's medical history.

The heterogeneous raw data may comprise medical images and/or pathological data and/or genetic data.

The recommendation may be a recommendation for treatment of a medical condition of the patient.

In summary, the method according to the invention may result in a model of the complex system, with which a user can interact. Based on the interaction of the user with the model, the method may update the model, as well as identify particular parts of the model that are relevant to the user. For example, the method may generate a model of a patient based on the patient's medical records. This model may be presented to a medical practitioner, who can then interact with the model. Such interaction may comprise querying the model about certain events in the patient's medical history and their connection to the patient's state of health. The interaction may also comprise feedback on hypotheses generated by the method about the patient's state of health.

The invention further provides a computing system comprising means for carrying out a method for modeling a complex system. The method may comprise one or more of the above-defined features.

The invention further provides a computer program comprising instructions, which, when the program is executed by a computer, cause the computer to carry out a method for modeling a complex system. The method may comprise one or more of the above-defined features.

### Brief description of the drawings

Advantageous embodiments will now be described in combination with the enclosed figures.
- Figure 1: schematically shows a system for modeling a complex system;
- Figure 2: schematically shows a chronological graphical representation of a complex system; and
- Figure 3: schematically shows a method according to the invention.

### Detailed description of the invention

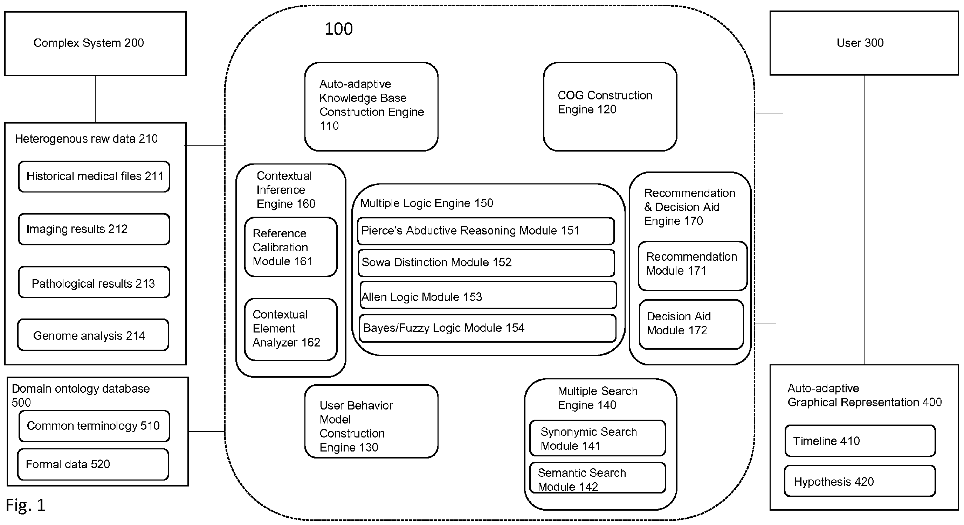

Figure 1 schematically shows a system 100 for modeling a complex system 200. In the illustrated embodiment, the complex system 200 is a human patient. Figure 1 further shows that heterogeneous raw data 210 is associated with the complex system 200. In the exemplary embodiment shown in Figure 1, the heterogeneous raw data 210 comprises a plurality of medical data, such as historical medical files 211, imaging data 212, pathological data 213 and genome analysis data 214. The imaging data 212 may comprise images obtained by X-ray imaging, magnetic resonance tomography (MRT) imaging, positron emission tomography (PET) imaging, or the like. The heterogeneous raw data 210 is stored in a variety of formats and accessible through one or more databases (no shown).

It can further be seen that a domain ontology database 500 is accessible by the system 100. The domain ontology database 500 provides access to common terminology for one or more domains. For example, in the illustrated embodiment, the domain ontology database 500 comprises common terminology 510 for the domains of medicine and human biology. Further, the domain ontology database 500 comprises data about the formal representations 520 of the one or more domains. For example, the ontology database 500 comprises information about how the components of the ontology interact with one another. Here, components of the ontology may comprise one or more of the following common ontology components: Individuals, classes, attributes, relations, function terms, restrictions, rules, axiom and events.

Figure 1 further shows that the system 100 comprises an Auto-adaptive Knowledge Base Construction Engine 110, a Conceptual Ontological Graph (COG) Construction Engine 120, a User Behavior Model Construction Engine 130, a Multiple Search Engine 140, comprising a Synonymic Search Module 141 and Semantic Search Module 142, and a Multiple Logic Engine 150. In the illustrated embodiment, the Multiple Logic Engine 150 comprises a Pierce's Abductive Reasoning Module 151, a Sowa's Distinction Module 152, an Allen's Logic Module 153, and a Bayes/Fuzzy Logic Module 154. The system 100 further comprises a Contextual Inference Engine 160 comprising Reference Calibration Module 161 and Contextual Element Analyzer 162, and a Recommendation & Decision Aid Engine 170 comprising a Recommendation Module 171 and a Decision Aid Module 172. Each of the respective modules of Multiple Search Engine 140, Multiple Logic Engine 150, Contextual Inference Engine 160 and Recommendation & Decision Aid Engine 170 provides one or more algorithms that allow programmatic application of their respective logic on data.

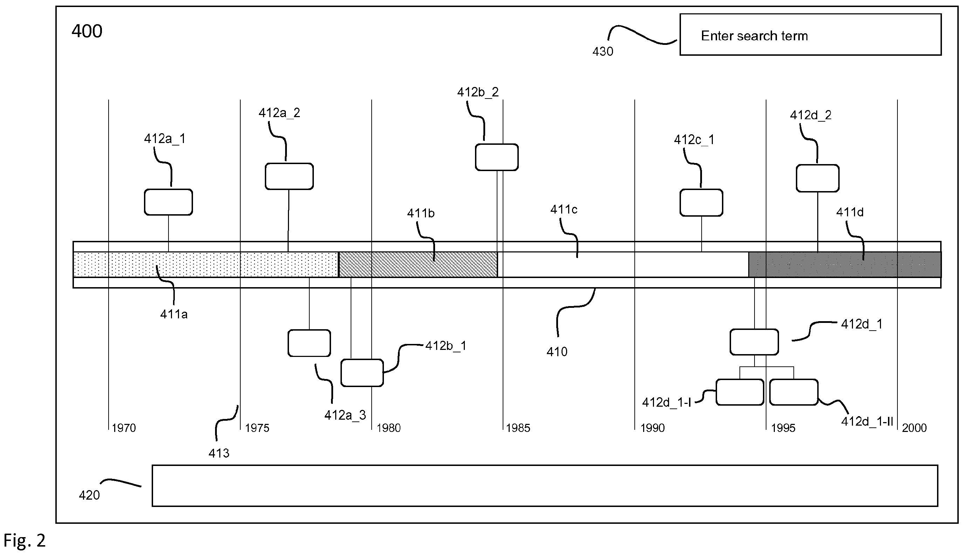

The system 100 is implemented in a computing device (not shown). The computing device comprises one or more processors, one or more memories, one or more interfaces for data input, and one or more interfaces for data output. The computing device comprises a network interface for accessing local or remote networks. The computing device further comprises an interface via which it can receive input from a user 300. The computing device comprises means to output an auto-adaptive graphical representation 400 of the complex system 200 to the user 300. The graphical representation comprises a timeline 410 of the complex system 200 and a hypothesis 420 concerning the complex system 200. The auto-adaptive graphical representation 400 of the complex system 200 will be described in more detail below with reference to Figure 2.

The system 100 is configured to retrieve one or more of heterogeneous raw data 210 of complex system 200. The retrieval of heterogeneous raw data 210 can, for example, be triggered by user 300 interacting with system 100. Advantageously, the system 100 can retrieve heterogeneous raw data 210 from a database. However, it is also possible that, for example, imaging results 212 are provided to system 100 via the one or more input interfaces of the computing device. For example, imaging results 212 which are only available in paper form can be scanned and transferred to system 100 via a data carrier such as a USB stick or via a network connection. It is also possible that imaging results 212 are scanned with a device directly connecting to the computing device in which system 100 is implemented. It is to be noted that this is not restricted to imaging results 212, but applies to all of heterogeneous raw data 210.

The system 100 is further configured to retrieve the common terminology 510 and data about formal representations 520 from domain ontology database 500.

The system 100 is further configured to use Contextual Inference Engine 160 to calibrate and contextualize domain reference 510 and 520 to adapt a general reference, in particular with respect to "local literature" concerning the complex system, using Reference Calibration Module 161 and to extract contextual elements from COGs and /or documents to constrain the sense inferring with Contextual Element Analyzer 162. Here, "local literature" specifically refers to a set of documents that is associated with the user or users of a specific instance of the system 100 and related to complex system 300. For example, if the system 100 is installed in a medical facility such as a hospital, "local literature" might refer to the entirety of patient records of the hospital. Reference to local literature allows the system 100 to more accurately determine the basis of the reference calibration procedure by identifying which terms are used in which context in the specific locale where system 100 is installed.

The system 100 is further configured to use Recommendation & Decision Aid Engine 170 using Recommendation Module 171 to suggest to the user further analysis and/or direct interaction, and a Decision aider 172 to guide the user concerning at least one hypothesis and the acts following this validation of hypothesis in the context.

Upon retrieving the one or more of heterogeneous raw data 210 of complex system 200, the common terminology 510 and data about formal representations 520, the system 100 is configured to use Reference Calibration Module 161 to calibrate the domain reference 520 based on a general corpus. For example, this process determines the contextual sense mining in the local medical literature. In the same way, system 100 is configured to use Contextual Analyzer 162 to extract and formalize from the documents related to COGs the contextual concepts to be added to the COGs in a later step.

The system 100 is further configured to use COG Construction Engine 120 to construct a plurality of COGs from the heterogeneous raw data 210. In more detail, the heterogeneous raw data 210 is analyzed by COG Construction Engine 120, which then proceeds to extract, clean and categorize raw text of the heterogeneous raw data 210 into the formal blocs "documents and acts" in order to structure the text for indexing. This indexed corpus forms the main base for further searches.

After that, Pierce's Abductive Reasoning Module 151 of Multiple Logic Engine 150 is employed in order to obtain "snippets" of knowledge and convert them into COGs and determine whether a particular COG is a state or an event thanks to Sowa's Distinction Module 152.

The system 100 is further configured to use Auto-adaptive Knowledge Base Construction Engine 110 to generate a knowledge base of complex system 200. In more detail, Auto-adaptive Knowledge Base Construction Engine 110 accesses Multiple Logic Engine 150 and employs Pierce's Abductive Reasoning Module 151 and Allen's Logic Module 153 in order to establish a chronological dependence and context for each of the COGs. Then, Auto-adaptive Knowledge Base Construction Engine 110 accesses Multiple Logic Engine 150 and employs Pierce's Abductive Reasoning Module 151 and Bayes/Fuzzy Logic Module 154 in order identify periods of the complex system 200, and associate the COGs with one or another periods. Thus, a "State-Hypothesis" is generated. In the illustrated embodiment, the periods generally correspond to a period in a life of a patient. Thereby, a knowledge base of complex system 200 is generated. At the same time, a timeline 410 of complex system 200 is created.

It is noted that system 100 is further configured to update an existing knowledge base of complex system 200 as new heterogeneous raw data 210 is received by system 100. In this case, the new heterogeneous raw data 210 is processed as described above in order to generate one or more COGs on the basis on the heterogeneous raw data 210, and to determine a chronological relation and context for each new COG. Then, Auto-adaptive Knowledge Base Construction Engine 110 accesses Multiple Logic Engine 150 and employs Pierce's Abductive Reasoning Module 151 and Bayes/Fuzzy Logic Module 154 on the entire set of COGs in order to update the knowledge base and timeline of the complex system 200.

After the knowledge base of complex system 200 has been generated, system 100 accesses Multiple Logic Engine 150 and employs Pierce's Abductive Reasoning Module 151 in order to formulate at least one hypothesis 420 concerning complex system 200. The hypothesis 410 may for example be a diagnosis, recommendation for treatment, or prognosis of the patient.

The system 100 further displays the timeline 410 and the hypothesis 420 to user 300 in an auto-adaptative graphical representation 400. The auto-adaptative graphical representation 400 allows user 300 to interact with the timeline 410 and the hypothesis 420. This will be described in more detail further below with reference to Figure 2.

User Behavior Model Construction Engine 130 is configured to analyze any interaction, i.e., acts and interactions, of user 300 with system 100, in particular any interaction of user 300 with timeline 410 and hypothesis 420. It is configured to access Multiple Logic Engine 150 and employ Pierce's Abductive Reasoning Module 151 in order to generate a model of user 300's behavior. User Behavior Model Construction Engine 130 may also access the COGs generated by COG Construction Engine 120 to identify previous interactions of user 300 with complex system 200.

The user model generated by User Behavior Model Construction Engine 130 can be accessed by Auto-adaptive Knowledge Base Construction Engine 110 and used as additional input for the generation and/or updating of the knowledge base and timeline 410 of complex system 200. The user model can further be utilized by system 100 when generating and/or updating hypothesis 420.

Multiple Search Engine 140 is configured to process search requests by user 300. In particular, Synonymic Search Engine 141 is configured to search for calibrated synonyms of given search terms within common terminology 510, and to then perform a search for all of these in the knowledge base. The search result is presented graphically, for example as a word cloud, to the user 300. Here, the word cloud represents the frequency of word co-occurrence sought with each synonym. User 300 may thus precisely refine their search due to the contextualization achieved through the use of the synonyms from uncalibrated common terminology 510. In particular, they may choose appropriate synonyms and combine them with suitable logical operators, e.g., "and", "or", "except", in order to construct a refined query.

The Semantic Search Engine 142 is configured to cross-references terms and the relation between terms of the search request with domain ontology database 500, and to search the knowledge base of complex system 200 for cross-references terms and their relations with the terms of the search request. In particular, Semantic Search Engine 142 enables user 300 to perform searches on the knowledge base of complex system in natural language. Semantic Search Engine 142 and Synonymic Search Engine 141 are further configured to display the search results to user 300. For example, Semantic Search Engine 142 makes it possible to search one or more terms concerning the medical state of a patient in his medical records.

Figure 2 schematically shows an auto-adaptive graphical representation 400 of a complex system 200. Auto-adaptive graphical representation 400 can, for example, be displayed to a user 300 of system 100 on a computer screen. It can be seen that the graphical representation comprises timeline 410. It is further illustrated that timeline 410 comprises several periods 411a to 411d. In particular, the periods 411a to 411d correspond to the periods of the complex system 200 as identified by Auto-adaptive Knowledge Base Construction Engine 110 of the system 100 described above.

It can further be seen that auto-adaptive graphical representation 400 comprises graphical elements 412a to 412d, which are associated with the periods 411a to 411d. The graphical elements 412a to 412d represent events of the complex system 200. For example, they may refer to certain diagnoses or examinations that have occurred in the respective associated period 411a to 411d of the complex system 200. The graphical elements 412a to 412d may be interactive graphical elements. For example, it is possible that the user 300 clicks on a graphical element 412b_1 and subsequently, system 100 provides information about the event that is represented by that graphical element 412b_1 to user 300. System 100 may further employ Synonymic Search Engine 141 and/or Semantic Search Engine 142 in order to automatically perform a search related to the event associated with graphical element 412b_1 or all elements of period 411b and present the search results to user 300. It can also be seen that graphical elements, such as graphical element 412d_1, depend on other graphical elements. This represents the fact that multiple events of the complex system 200 may have a common effect on complex system 200. In the illustrated case, graphical elements 412d_1-I and 412d_1-II are connected in this way to graphical element 412d_1.

Figure 2 further illustrates that the auto-adaptive graphical representation 400 comprises vertical indicators 413. The vertical indicators 413 indicate a timescale on which timeline 410 is displayed. In the illustrated example, the distance between two vertical indicators 413 represents five years of time. User 300 may zoom auto-adaptive graphical representation 400 in order to fit a given time window in the display. The time window can be of arbitrary length. The time window may, for example, be indicated by user 300 indicate with a suitable input device, such as a mouse or their finger when using a touchscreen device (not shown).

Further, it can be seen in Figure 2 that auto-adaptive graphical representation 400 comprises a display of hypothesis 420 generated by system 100. The display of hypothesis 420 is also used to display recommendations and decision aids provided by Recommendation Module 171 and Decision Aid Module 172. The display of hypothesis 420 is updated based on user interaction with the timeline 410 and graphical elements 412a to 412d. It is also updated based on the availability of new raw heterogeneous data 210 about the complex system 200, new logs and acts of user 300, and corresponding updates to the knowledge database, as described above with reference to Figure 1. Display of hypothesis 420 may, for example, display a recommendation to user 300 that the patient is due for a checkup examination, and which examinations should be performed during that checkup. Alternatively, if new raw heterogeneous data 210 has been provided to system 100, for example the blood culture results of a recent examination, display of hypothesis 410 might indicate a first diagnosis based on the updated knowledge base.

Finally, Figure 2 shows that auto-adaptive graphical representation 400 comprises a search box 430. Search box 430 may be used by user 300 to input search queries to system 100. Upon detection that a search query has been entered into search box 430, system 100 employs Multiple Search Engine 140 in order to automatically perform a search related to the query as described above with reference to Figure 1. The search query and/or the queries results may be used to update the hypothesis 420 and its display.

While not shown explicitly in Figure 2, as described above with reference to Figure 1, system 100 employs User Behavior Model Construction Engine 130 to monitor interactions of user 300 with the graphical representation 400, in particular timeline 410, graphical elements 412a to 412d, and search box 430. Based on these interactions, User Behavior Model Construction Engine 130 creates a user model for user 300, or, if a user model for user 300 already exists, updates that user model accordingly.

By way of example, the auto-adaptive graphical representation 400 illustrated in Figure 2 shows the timeline 410 of a female patient's medical history. The patient was born prior to 1970, and period 411a indicates the patient's childhood and adolescence. Graphical element 412c indicates that an X-ray imaging examination occurred during the period 411a, in particular during her late adolescence. By clicking on graphical element 412a_3, user 300 may be presented with information associated with said X-ray imaging, for example that it was caused by an accident resulting in a broken leg. User 300 may further be presented with further data relating to this, for example, how many other X-ray imaging examinations have been performed on the patient, in which period they occurred and if they were performed on the same body area of the patient. The user 300 may further be presented with additional data relating the broken leg, e.g., if surgery was performed on the leg due to this accident, or if any surgery was performed on the leg before or after the accident.

Period 411b indicates (approximately) the patient's early adult life. It can be seen that graphical element 412b is associated with the near end of period 411b and the beginning of period 411c. Here, graphical element 412b represents first signs of pregnancy and the birth of the patient's first child. Thus, it can be seen that system 100 has determined that the patient's new state of life belongs to the next period.

It can also be seen that graphical element 412b_1 is close to the transition line from period 411a to 411b. This is because the system 100 has determined that period 411a represents the patient's childhood and adolescence. The transition to period 411b, representing early adulthood was ended when a gynecological examination confirmed the condition of her genital organ, represented by graphical element 412b_1. The start line of period 411b is, thus, determined to be associated with an event which took place in this period

As shown, the beginning of new periods in the patient's adult life will generally be caused by an external event, as described with reference to graphical element 412b_1 above. However, the respective graphical element must not necessarily line up with the beginning of the period. This can be seen with graphical elements 412b_1 and 412d_1. Graphical element 412d_1 for example, marks a transition between periods 411c and 411d. For example, graphical element 412d_1 and the graphical elements connected to it may indicate examinations that indicated an illness, such as diabetes. However, system 100 has determined that the diabetes occurred in the patient before the time of graphical element 412d_1, i.e., before examinations, symptoms, and diagnosis related to the diabetes were actually performed. This inference is made by system 100 based on the physician's diagnosis informing the date of onset of the outbreak of the disease. As such, period 411c starts already before the time indicated by graphical element 412d_1.

In the illustrated embodiment, display of hypothesis 420 may, for example, display a recommendation to user 300 that the patient is due for a checkup examination, and that in particular a blood count should be performed. User 300 could then, for example, enter the search query "blood count" into search box 430, and system 100 would, by means of Multiple Search Engine 140, retrieve results from the knowledge base and present them to user 300, as described above with reference to Figure 1.

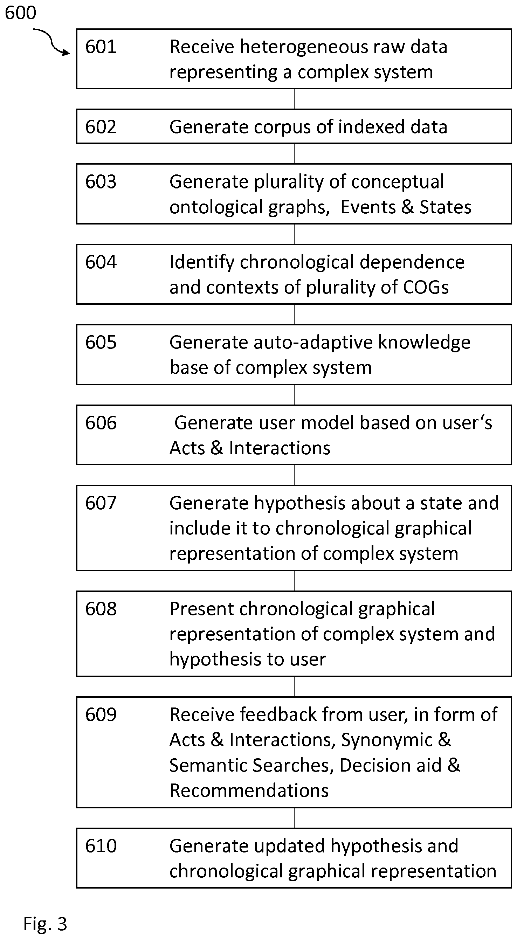

Figure 3 schematically shows a flow diagram of a method 600 according to the invention. In step 601, system 100 receives heterogeneous raw data 210 representing a complex system 200.

In step 602, a corpus of indexed data is generated by system 100 by applying rules and constraints from one or more domain ontologies to the heterogeneous raw data 210. Step 602 may comprise the system 100 accessing one or more ontology databases 500 which store common calibrated terminology for one or more domains. Step 602 comprises analyzing the heterogeneous raw data 210 by COG Construction Engine 120 and converting it into a corpus of indexed data to be used by Multiple Search Engines 140 and COG Construction Engine 120

In step 603, a plurality of conceptual ontological graphs is generated by system 100. For this, system 100 employs COG Construction Engine 120 to construct a plurality of COGs from the heterogeneous raw data 210.. Step 603 further comprises COG Construction Engine 120 accessing Multiple Logic Engine 150 and employing Pierce's Abductive Reasoning Module 151 and Sowa's Distinction Module 152 in order to process the corpus of indexed data and generate the plurality of COGs. Here, the COG Construction Engine 120 uses Pierce's Abductive Reasoning Module 151 to identify and compile related data from the raw textual data into respective COGs, and it uses Sowa's Distinction Module 152 to determine whether a particular COG is a state or an event.

In step 604, the chronological dependence and context of COGs is identified by system 100. For this, Auto-adaptive Knowledge Base Construction Engine 110 accesses Multiple Logic Engine 150 and employs Pierce's Abductive Reasoning Module 151 and Allen's Logic Module 153 in order to establish a chronological dependence and context for each of the COGs. Then, Auto-adaptive Knowledge Base Construction Engine 110 accesses Multiple Logic Engine 150 and employs Pierce's Abductive Reasoning Module 151 and Bayes/Fuzzy Logic Module 154 in order identify periods of the complex system 200 and associate the COGs with one or more periods.

In step 605, an auto-adaptive knowledge base of complex system 200 is generated by system 100 via Auto-adaptive Knowledge Base Construction Engine 110.

In step 606, a user model is generated based on the user's Acts & Interactions. In particular, system 100 may employ User Behavior Model Construction Engine 130 in order to analyze user feedback, and construct a user model for user 300 or update an existing user model for user 300.

In step 607, a hypothesis 420 about the current state of complex system 200 is generated by system 100. This step further comprises generation of a chronological graphical representation 400, comprising a timeline 410 of the complex system 200 to be displayed to user 300 in the next step 608. For this, the system 100 employs Pierce's Abductive Reasoning Module 151 and Bayes/Fuzzy Logic Module 154.

In step 608, the auto-adaptive chronological graphical representation 400, including timeline 410 and display of hypothesis 420 is displayed to a user 300.

In step 609, feedback on the auto-adaptive chronological representation 400 and/or timeline 410 and/or display of hypothesis 420 and/or the results of searches and/or recommendations and/or decision aids from user 300 is received by system 100.

In step 610, an updated hypothesis and auto-adaptive chronological graphical representation 400 is generated by system 100 based on the received user feedbacks, the user model, and new raw data 210. The updated hypothesis and auto-adaptive chronological graphical representation 400 is then presented to user 300.

Steps 609 and 610 may be repeated multiple times.

Although the previously discussed embodiments and examples of the present invention have been described separately, it is to be understood that some or all of the above-described features can also be combined in different ways. The above discussed embodiments are particularly not intended as limitations, but serve as examples, illustrating features and advantages of the invention.

## Claims

1. A computer-implemented method for modeling a complex system, CS, the method comprising:
receiving heterogeneous raw data representing the CS;
applying rules and constraints from one or more domain ontologies to the heterogeneous raw data to obtain a corpus of categorized and terminologically indexed data;
applying a first set of algorithms to the corpus to obtain a plurality of conceptual ontological graphs, COGs, wherein each of the plurality of COGs is categorized as either a state or an event;
applying a second set of algorithms to the COGs to identify a chronological dependence of the classified states and events, and to identify a context for each of the classified states and events;
applying a third set of algorithms to the COGs to consolidate the classified states and events such that the CS is **characterized by** one or more periods, wherein one period is **characterized by** a consolidated state and one or more events associated with the consolidated state, in order to generate a knowledge base of the CS;
applying a fourth set of algorithms to the knowledge base to obtain at least one hypothesis of how at least one period of the CS relates to at least one event;
generating, using the knowledge base, the at least one period, and the at least one hypothesis, a chronological graphical representation of the complex system;
presenting the chronological graphical representation of the complex system and the at least one hypothesis to a user;
receiving interaction and feedbacks on at least the at least one period and at least one hypothesis from the user; and
applying a fifth set of algorithms to the feedback received from the user and the chronological graphical representation of the complex system to generate an updated knowledge base, an updated chronological graphical representation, and an updated hypothesis of the complex system.

2. The method of claim 1, wherein
the first, second, third, fourth and fifth set of algorithms are at least partly based on Peirce's Abductive reasoning logic;
the first set of algorithms is further based Sowa's distinction of processes;
the second set of algorithms is further based on Allen's interval algebra; and
the third set of algorithms is further based on Bayesian, fuzzy, and Allen's logic.

3. The method of any of the preceding claims, further comprising generating a user behavioral model, UBM, based on the interactions of the user with the complex system.

4. The method of claim 4, further comprising adapting the chronological graphical representation of the complex system based on the generated UBM.

5. The method of claims 3 or 4, further comprising generating at least one recommendation and /or decision aid for interaction of the user with a current state of the complex system, based on the updated hypothesis and the generated UBM.

6. The method according to any of the preceding claims, wherein the complex system is a patient's medical history.

7. The method according to claim 6, wherein the heterogeneous data comprises medical images and/or pathological data and/or genetic data.

8. The method according to any of claims 6 or 7, wherein the recommendation is a recommendation for treatment of a medical condition of the patient.

9. A computing system comprising means for carrying out the method according to of any of the claims 1 to 8.

10. A computer program comprising instructions which, when the program is executed by a computer, cause the computer to carry out the method according to of any of the claims 1 to 8.
